# EUROPEAN PATENT APPLICATION

(11) **EP 2 857 572 A1**
(43) Date of publication of application: **08.04.2015**
(21) Application number: 14182813.7
(22) Date of filing: 29.08.2014
(51) Int. Cl.: D06F 39/00, A47L 15/42, C02F 1/42

(54) **Softening apparatus and washing machine including the same**

(30) Priority: 02.10.2013 KR 20130117616; 20.01.2014 KR 20140006673
(71) Applicant: Samsung Electronics Co., Ltd, Gyeonggi-do 443-742 (KR)
(72) Inventor: Lim, Chang Bae, Gyeonggi-do (KR); Park, Hee Jin, Gyeonggi-do (KR); Jeong, In Jo, Gyeonggi-do (KR); Hamanaka, KATSUHIKO, Gyeonggi-do (KR)
(74) Representative: Taor, Simon Edward William

(57) **Abstract**

Electrochemical softening apparatus (100) regenerating a zeolite compound that has been used to perform ion exchange using hydrogen ions (H⁺) generated using an electrochemical method such that the zeolite compound is repeatedly used, whereby said electrochemical softening apparatus (100) comprises a regeneration unit (130) to generate regeneration water containing hydrogen ions (H⁺) and a softening unit (120), including an ion exchange body (121) regenerated by the regeneration water, to convert raw water containing a hardness component into soft water containing hydrogen ions (H⁺).

## Description

Embodiments of the present disclosure relate to a softening apparatus and a washing machine including a softening apparatus that simultaneously performs softening and washing (sterilization, descaling, etc.) and a washing machine including the same.

When detergent is used to remove non-polar contaminants from electric home appliances (a washer, a dishwasher, etc.) using water, cleaning performance may be deteriorated due to hardness of the water and the electric home appliances may be contaminated due to microorganisms propagating in the water and a scale component.

In order to prevent cleaning performance from being deteriorated due to hardness of the water, a heater may be used to increase solubility of the detergent, a hardness component may be removed using an ion exchange method, or electrochemical capacitive deionization (CDI) using electrostatic attractive force of an ion component may be applied. However, these methods do not fundamentally remove a hardness component (e.g. Ca²⁺ or Mg²⁺) with the result that the hardness component may deposited on an object to be washed or a complicated system may be used to remove the hardness component. In this case, however, material costs may be increased and high energy may be needed. Ion exchange resin using an ion exchange method is relatively inexpensive and convenient. When the ion exchange resin is regenerated for repetitive use, however, a high-concentration sodium chloride solution (NaCl) is used. As a result, actual application to the system is limited due to user inconvenience and environmental regulations due to regenerated waste water.

In order to prevent the cleaning system from being contaminated due to microorganisms, various methods, such as high-temperature sterilization, decolorant ion sterilization, and negative ion sterilization, may be used. However, these methods may require high energy and cause user inconvenience due to use of additional consumable chemicals. In addition, actual application to the system is limited due to environmental regulations.

For contamination due to the scale component, there are insufficient solutions.

It is an aspect to provide an electrochemical softening apparatus that is relatively convenient and inexpensive and a washing machine including the same. It is an aspect to provide a softening apparatus that regenerates zeolite that has been used to perform ion exchange using hydrogen ions (H⁺) generated using an electrochemical method such that the zeolite is repeatedly used and a washing machine including the same.

It is an aspect to provide a softening apparatus designed such that hydrogen ions exchanged with a hardness component during a re-softening process are used to remove contaminants due to microorganisms and a scale component and a washing machine including the same.

Additional aspects will be set forth in part in the description which follows and, in part, will be obvious from the description, or may be learned by practice of the disclosure. In accordance with one aspect, a softening apparatus includes a regeneration unit to generate regeneration water containing hydrogen ions (H⁺) and a softening unit, including an ion exchange body regenerated by the regeneration water, to convert raw water containing a hardness component into soft water containing hydrogen ions (H⁺).

The regeneration unit may electrolyze water to generate the hydrogen ions (H⁺). The water may include soft water.

The regeneration unit may include a housing forming an external appearance and an electrode provided in the housing.

The ion exchange body may be coupled to one side of the electrode via a binder such that the regeneration unit and the softening unit are integrally formed. The binder may include at least one selected from a group consisting of an inorganic binder and a porous binder.

The regeneration unit may include a cyclone type housing, a cylindrical anode provided in the housing, and a cathode disposed on a central axis of the anode, and the ion exchange body may be disposed between the anode and the cathode.

The hardness component of the raw water may be adsorbed by the ion exchange body of the softening unit and, at the same time, hydrogen ions (H⁺) may be separated from the ion exchange body to soften the raw water.

The hydrogen ions (H⁺) contained in the regeneration water supplied from the regeneration unit may be adsorbed by the ion exchange body of the softening unit and, at the same time, a hardness component may be separated from the ion exchange body to regenerate the ion exchange body.

The ion exchange body may include at least one selected from a group consisting of zeolite, activated carbon, platinum (Pt), titanium (Ti), titanium oxide (TiO₂), carbon black, and manganese (Mn).

The ion exchange body may be of a bead type or a powder type. The softening apparatus may further include a heater to heat water supplied to at least one selected from between the softening unit and the regeneration unit.

In accordance with an aspect, a washing machine includes a washing device, a softening apparatus, and a controller to control operation of the washing device and the softening apparatus, wherein the softening apparatus includes a regeneration unit to generate regeneration water containing hydrogen ions (H⁺) and a softening unit, including an ion exchange body regenerated by the regeneration water, to convert raw water containing a hardness component into soft water containing hydrogen ions (H⁺).

When the washing device is operated in a sterilization mode, the controller may control the softening unit to generate soft water containing hydrogen ions (H⁺) such that the soft water is used to sterilize or descale the washing device.

The washing machine may further include a detergent supply device to supply detergent to the soft water discharged from the softening unit.

When the washing device is operated in a washing mode, the controller may control the soft water discharged from the softening unit to be mixed with the detergent supplied from the detergent supply device such that the mixture is provided to the washing device.

When the washing device is operated in a regeneration mode, the controller may control the regeneration unit to generate regeneration water containing hydrogen ions (H⁺) and to supply the regeneration water to the softening unit such that the ion exchange body is regenerated.

The washing machine may further include a channel unit to guide soft water generated by the softening unit or condensed water containing a hardness component.

The washing machine may further include a hardness sensor to sense hardness of the soft water discharged from the softening unit. When the output of the hardness sensor reaches predetermined first reference hardness, the controller may control the regeneration mode to be executed.

The washing machine may further include an electric conductivity sensor to sense electric conductivity of the soft water discharged from the softening unit. When the output of the electric conductivity sensor reaches predetermined second reference conductivity, the controller may control the regeneration mode to be executed.

The washing machine may further include a flow rate sensor to sense flow rate of the soft water discharged from the softening unit. When the output of the flow rate sensor reaches predetermined third reference flow rate, the controller may control the regeneration mode to be executed.

The controller may control the regeneration mode to be executed during the washing mode or the sterilization mode of the washing device.

The regeneration unit may include a housing forming an external appearance and an electrode provided in the housing and the ion exchange body may be coupled to one side of the electrode via a binder such that the regeneration unit and the softening unit are integrally formed.

The binder may include at least one selected from a group consisting of an inorganic binder and a porous binder.

The washing machine may further include a heater to heat water supplied to at least one selected from between the softening unit and the regeneration unit.

In accordance with an aspect, an operation method of a washing machine including a washing device and a softening apparatus comprising an ion exchange body having hydrogen ions (H⁺) adsorbed thereby includes supplying raw water containing a hardness component to the softening apparatus to generate soft water containing hydrogen ions and providing the generated soft water to the washing device to wash or sterilize the washing device.

The operation method may further include supplying detergent to the generated soft water and providing the soft water containing the detergent to the washing machine to wash the washing device.

The operation method may further include heating the raw water containing the hardness component using a heater.

The operation method may further include determining whether the softening apparatus is to be regenerated and, determining that the softening apparatus is to be regenerated, regenerating the softening apparatus.

The determining whether the softening apparatus is to be regenerated may include at least one selected from among sensing a hardness value of the generated soft water, sensing electrical conductivity of the generated soft water, and total flow rate of the soft water generated by the softening apparatus.

These and/or other aspects will become apparent and more readily appreciated from the following description of the embodiments, taken in conjunction with the accompanying drawings of which:
FIG. 1 is a view showing construction of a softening apparatus according to an embodiment;
FIG. 2 is a view showing a softening process of the softening apparatus according to the embodiment;
FIG. 3 is a view showing a regeneration process of the softening apparatus according to the embodiment;
FIG. 4 is a view showing the softening and regeneration processes performed in

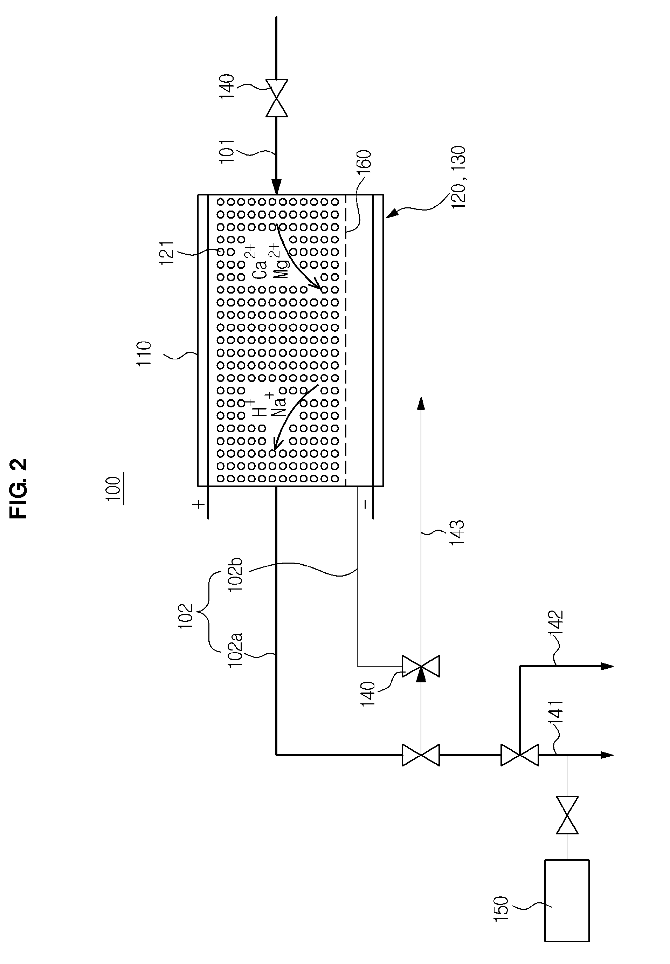

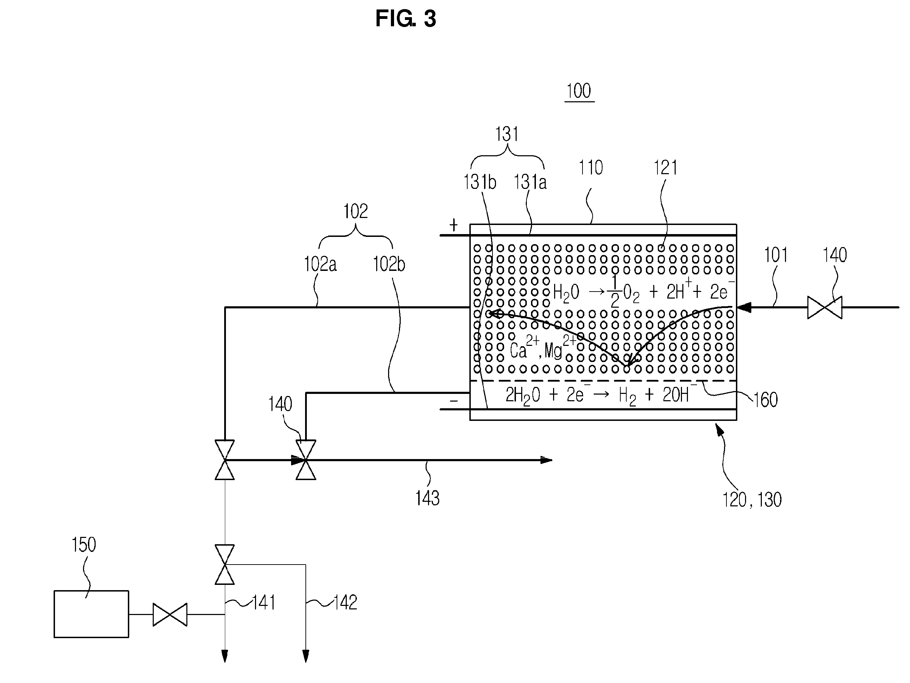

FIGS. 2 and 3 as a chemical reaction formula;

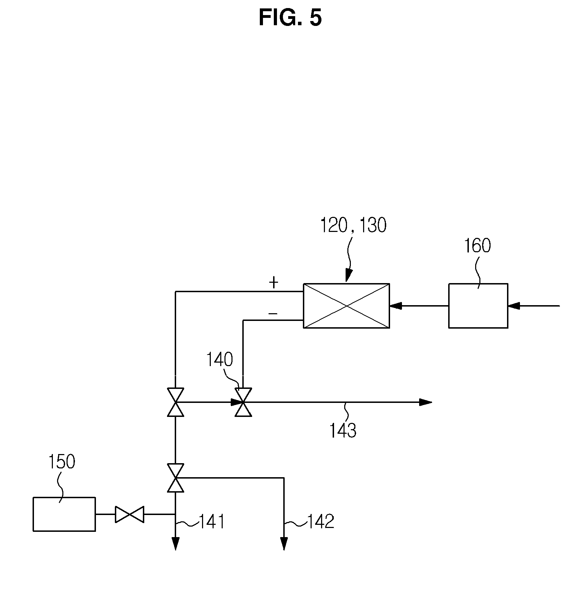

FIG. 5 is a view showing construction of a softening apparatus including a heater according to an embodiment;

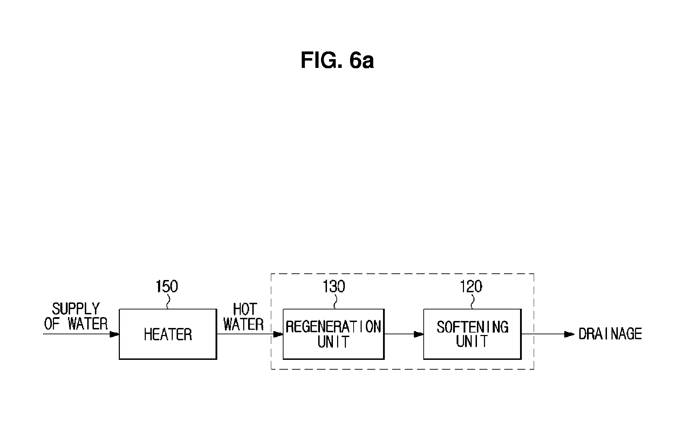

FIGS. 6A to 6C are views showing positions where the heater may be installed in the softening apparatus shown in FIG. 5;

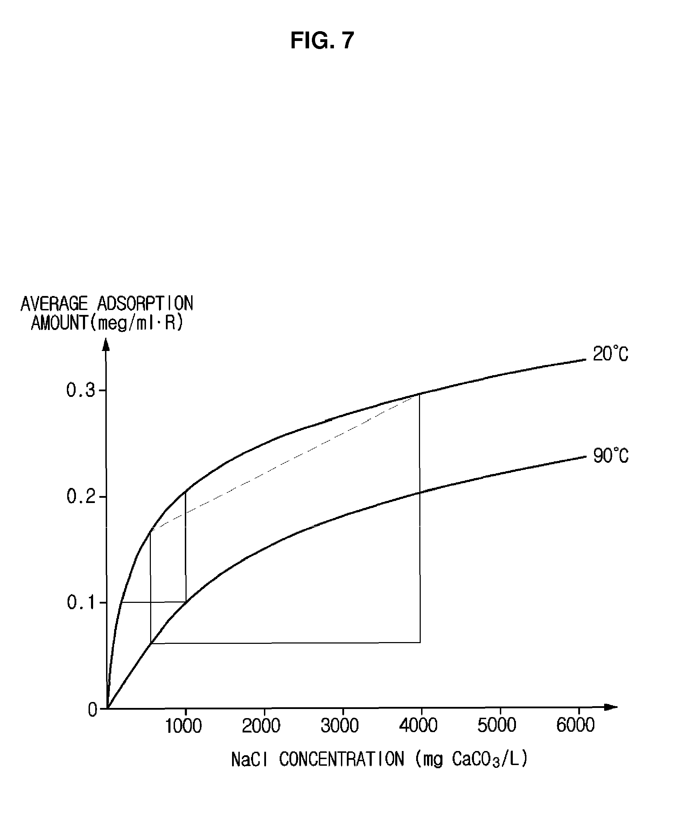

FIG. 7 is a graph showing the average adsorption amount of sodium ions based on concentration of sodium chloride per temperature;

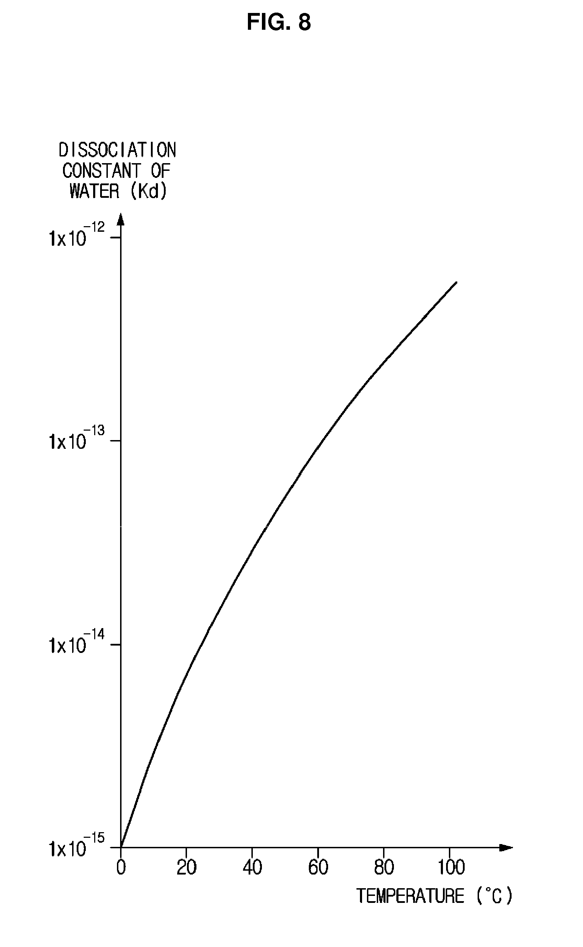

FIG. 8 is a graph showing a dissociation constant of water based on temperature;

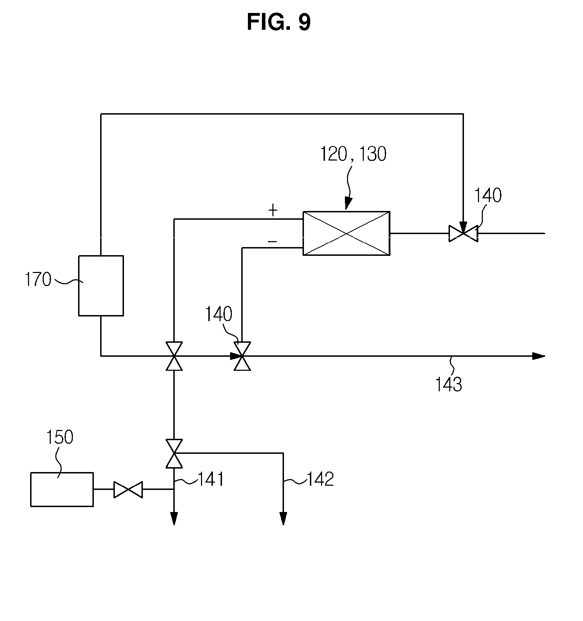

FIG. 9 is a view showing construction of a softening apparatus including a storage tank according to an embodiment;

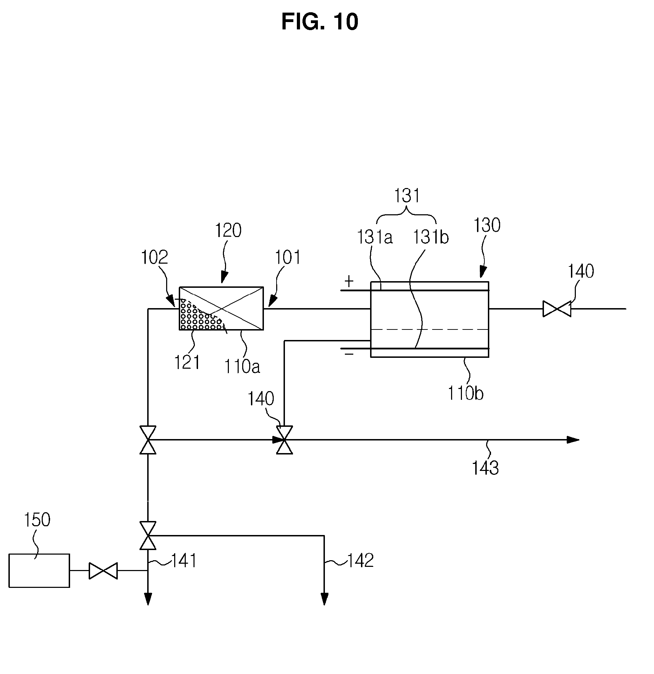

FIG. 10 is a view showing a softening apparatus including a softening unit and a regeneration unit, which are separated from each other, according to an embodiment;

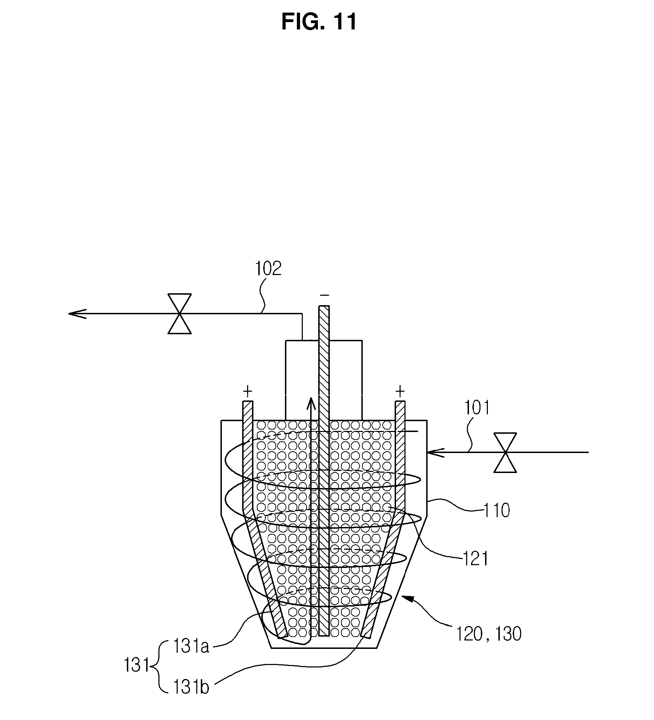

FIG. 11 is a view showing a cyclone type softening apparatus according to an embodiment;

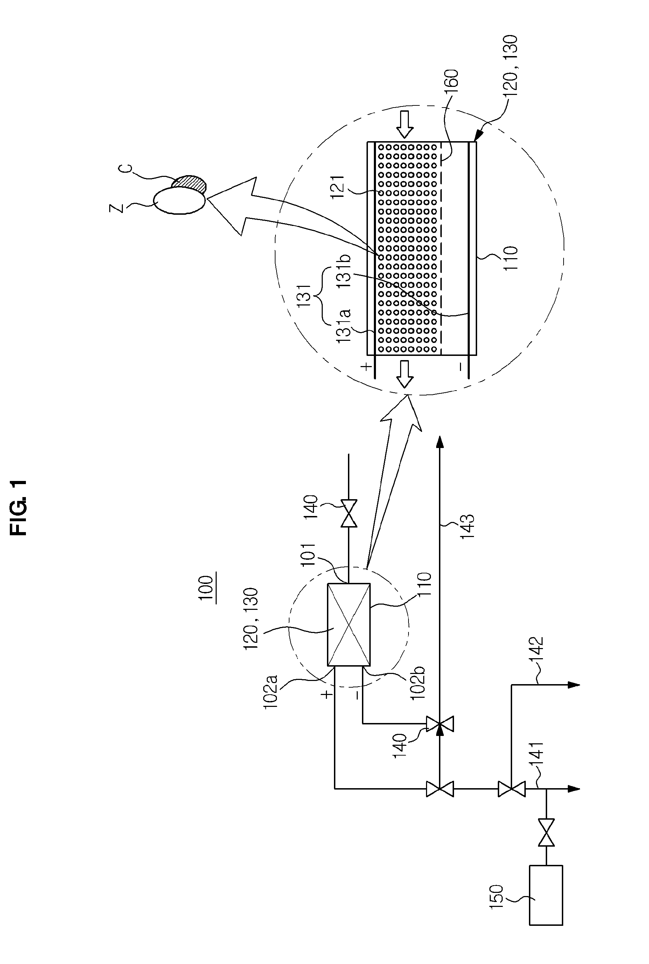

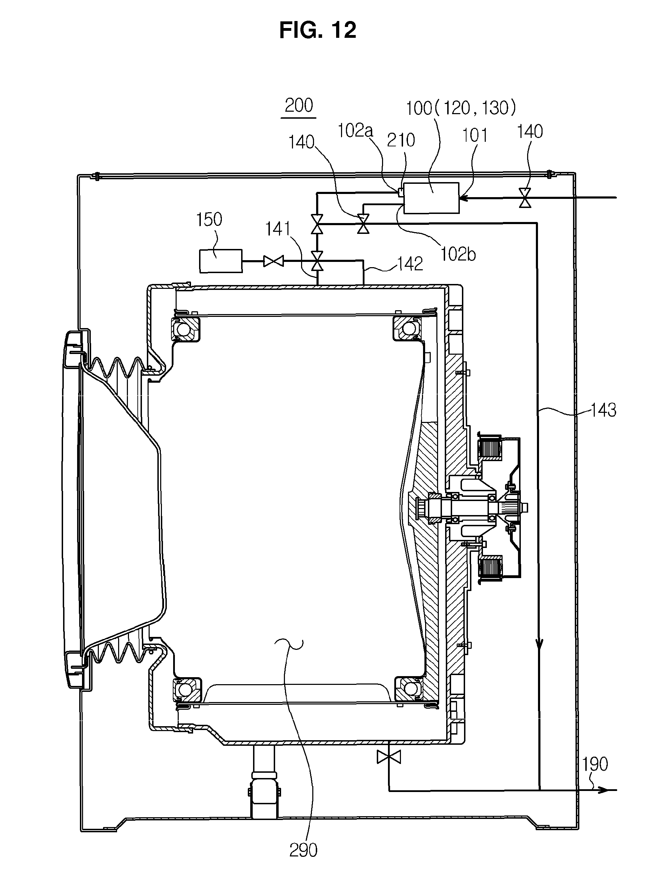

FIG. 12 is a view showing a washing machine including the softening apparatus of FIG. 1;

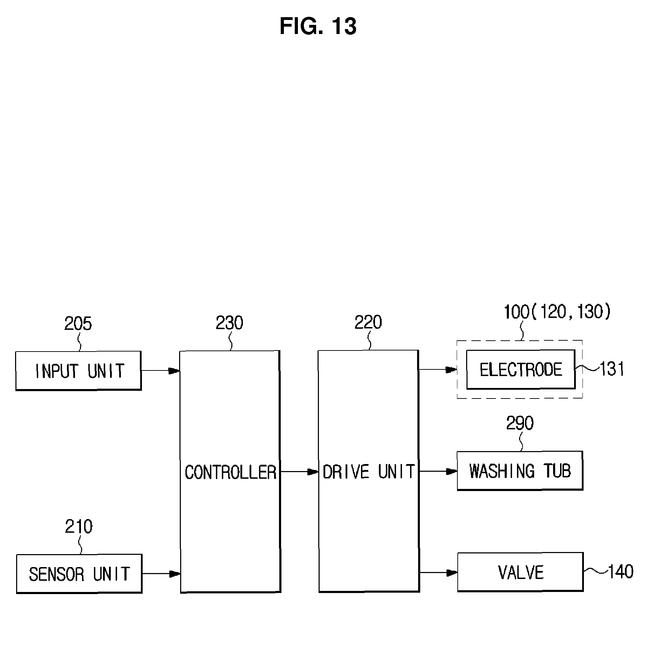

FIG. 13 is a control block diagram of the washing machine shown in FIG. 12; and

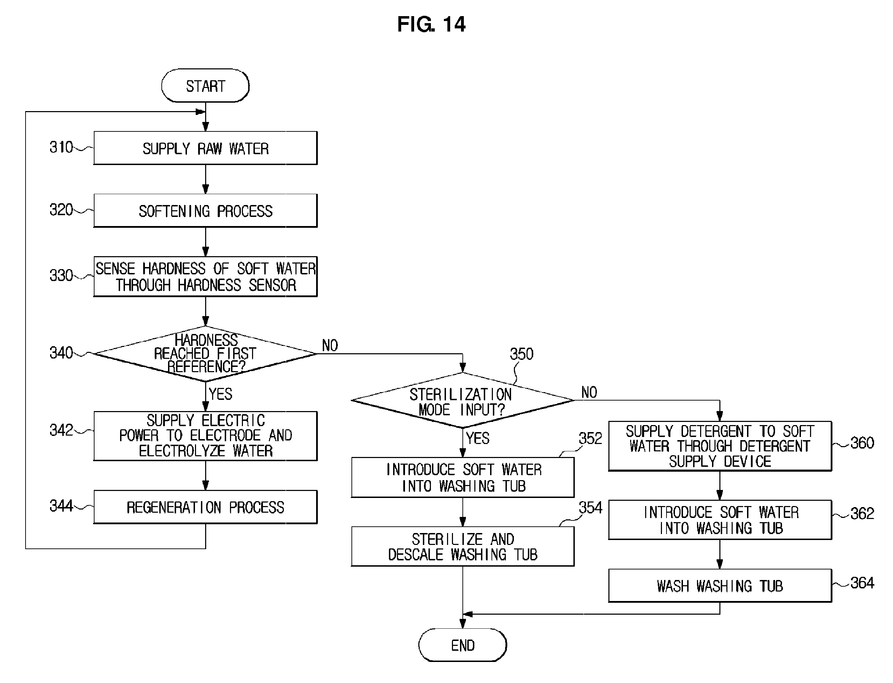

FIG. 14 is a flowchart showing a control process of a washing machine according to an embodiment.

Reference will now be made in detail to the embodiments, examples of which are illustrated in the accompanying drawings, wherein like reference numerals refer to like elements throughout.

Embodiments relate to a softening apparatus that softens raw water containing a hardness component and a washing machine including the same. In this specification, supply water containing a hardness component introduced into the softening apparatus is referred to as raw water, raw water, from which the hardness component has been removed, discharged from a softening unit is referred to as soft water, supply water having high concentration of hydrogen ions (H⁺) electrolyzed and supplied to an ion exchange body is referred to as regeneration water, and regeneration water having high concentration of a hardness component through a regeneration process is referred to as condensed water for the convenience of description. The hardness component may include positive ions, such as calcium ions (Ca²⁺) and magnesium ions (Mg²⁺), having positive charges. Hereinafter, a description will be given on the assumption that the hardness component includes calcium ions and magnesium ions for the convenience of description.

Hereinafter, embodiments will be described in detail with reference to the accompanying drawings. FIG. 1 is a view showing construction of a softening apparatus 100 according to an embodiment.

Referring to FIG. 1, the softening apparatus 100 includes a housing 110 having an inlet port 101 and an outlet port 102, a softening unit 120 having an ion exchange body 121 to convert raw water into soft water, a regeneration unit 130 to regenerate the ion exchange body 121 using hydrogen ions (H⁺) generated during electrolysis of water, and channel units 141, 42, and 143 to guide soft water discharged from the softening unit 120 or condensed water discharged from the regeneration unit 130. In addition, the softening apparatus 100 may further include a detergent supply device 150 to supply detergent to the soft water discharged from the softening unit 120. The softening unit 120 and the regeneration unit 130 are provided for softening and regeneration, respectively. The softening unit 120 and the regeneration unit 130 may be separated from each other. In this embodiment, however, a softening and regeneration element is integrally formed in the housing 110. Consequently, the softening and regeneration device can be referred to as the softening unit 120 when generating soft water through a softening process and as regeneration unit 130 when performing a regeneration process.

Hereinafter, the respective elements of the softening apparatus 100 will be described in more detail.

The housing 110 includes an inlet port 101 connected to a raw water pipe to allow raw water to be introduced therethrough and an outlet port 102 connected to a water discharge pipe to allow soft water to be discharged therethrough. The inlet port 101 may be formed at a central axis of the top of the housing 110 and the outlet port 102 may be formed at a central axis of the bottom of the housing 110. The inlet port 101 and the outlet port 102 are provided with valves 140 to allow or block flow of raw water to be introduced into the inlet port 101 and soft water to be discharged to the inlet port 101. During operation of the softening apparatus 100, the valves 140 may be controlled to properly adjust introduction of raw water and discharge of soft water.

The softening unit 120 is provided in the housing 110. The softening unit 120 is an element to remove a hardness component from raw water introduced through the inlet port 101 of the softening apparatus 100 to soften the raw water. The softening unit 120 softens water based on ion exchange capability of the ion exchange body 121. In embodiments of the invention, the softening unit 120 may be integrally formed with or may be separated from the regeneration unit 130. In FIG. 1, the softening unit 120 is integrally formed with the regeneration unit 130.

The ion exchange body 121 may be, for example of a bead type or a powder type, but embodiments of the invention are not limited to those types. The ion exchange body 121 may fill the softening unit 120. The ion exchange body 121 may be coupled to one side of an electrode 131, for example the surface of an anode via a binder. At least one selected from a group consisting of an inorganic binder and a porous binder may be used as the binder to increase the ion exchange amount of the ion exchange body 121.

A bead type zeolite compound is obtained by adding a binder to powder type zeolite particles (Z) and forming the powder type zeolite particles in a spherical shape. Water easily passes through the bead type zeolite compound since gaps among the particles are large. However, the bead type zeolite compound has a smaller specific surface area than a powder type zeolite compound with the result that softening performance per unit weight may be deteriorated. The powder type zeolite compound has a large specific surface area with the result that softening performance per unit weight is excellent. However, gaps among the particles are small. When water passes through the powder type zeolite compound, therefore, differential pressure may greatly increase.

Consequently, a proper sized zeolite compound may be used for the above reasons. Furthermore, activated carbon (C) may be coupled to the zeolite compound or the housing 110 may be designed to have a cyclone structure. In FIGS. 1 and 2, the ion exchange body 121 is formed by coupling the activated carbon (C) to the zeolite compound.

In addition, the ion exchange body 121 may include at least one selected from a group consisting of an ion exchange material having zeolite, ion exchange resin, ion exchange thin film, ion exchange fiber, and at least one inorganic metal ion selected from a group consisting of aluminum (Al), zirconium (Zr), and silicon (Si) as central atoms and an ion exchangeable site on the surface thereof, a material formed by introducing a functional group or a polymer compound to the surface of zeolite or ion exchange resin, a compound formed by introducing an ion exchange group including zeolite to at least one selected from a group consisting of platinum (Pt), titanium (Ti), titanium oxide (TiO₂), manganese (Mn), carbon black, and zeocarbon.

The regeneration unit 130 is an element to electrolyze raw water to remove hard impurities from the ion exchange body 121. More specifically, the regeneration unit 130 supplies hydrogen ions (H⁺) generated during electrolysis of water to the ion exchange body 121 to regenerate the ion exchange body 121.

The regeneration unit 130 includes an electrode 131 to electrolyze raw water. The electrode 131 includes an anode 131a and a cathode 131b spaced apart from the anode 131a. At least one anode 131a and at least one cathode 131b may be provided. For example, the anode 131a and the cathode 131b each may be formed in the shape, for example, of a circular electrode, a bar electrode, or a plate electrode.

In FIG. 1, the anode 131a and the cathode 131b each are formed in the shape of a plate electrode for the convenience of description. Alternatively, the anode 131a may be formed in the shape of a circular electrode such that the anode 131a extends in a longitudinal direction and the cathode 131b may be formed in the shape of a bar electrode such that the cathode 131b is disposed inside the anode 131a. In addition, pluralities of anodes 131a and cathodes 131b may be provided such that the anodes 131a and the cathodes 131b may be alternately arranged.

In addition, the regeneration unit 130 may include a diaphragm 160 disposed between the anode 131a and the cathode 131b to selectively transmit ions. The diaphragm 160 may include at least one selected from a group consisting of non-woven fabric, membrane, and ion exchange film.

As needed, a plurality of regeneration units 130 may be provided to constitute a regeneration module. In this case, regeneration may be more rapidly and effectively performed.

The channel units 141, 42, and 143 guide soft water or condensed water discharged from the softening unit 120 or the regeneration unit 130. Referring to FIG. 1, acid soft water obtained by removing a hardness component from raw material and condensed water containing a hardness component separated from the ion exchange body 121 may be discharged from the anode 131a side based on the diaphragm 160 and alkali water may be discharged from the cathode 131b side. The channel units 141, 42, and 143 guide soft water discharged from the softening apparatus 100 such that the soft water is properly supplied as described above. Components of soft water and condensed water will be explained in detail when operation of the softening apparatus 100 is described below.

The channel units 141, 142, and 143 may include a first channel unit 141, a second channel unit 142, and a third channel unit 143. The first channel unit 141 guides acid soft water to be supplied to a supply unit of the detergent supply device 150. The second channel unit 142 guides acid soft water to be moved to a position where sterilization and descaling are performed. The third channel unit 143 guides condensed water and alkali water to be discharged outside.

The detergent supply device 150 is provided in the vicinity of the first channel unit 141. The softening apparatus 100 supplies softened wash water to an apparatus connected to the softening apparatus 100 or including the softening apparatus 100. The softening apparatus 100 may supply detergent to soft water through the detergent supply device 150.

Hereinafter, softening and regeneration processes and principles of the softening apparatus 100 with the above-stated construction according to the embodiment will be described in detail.

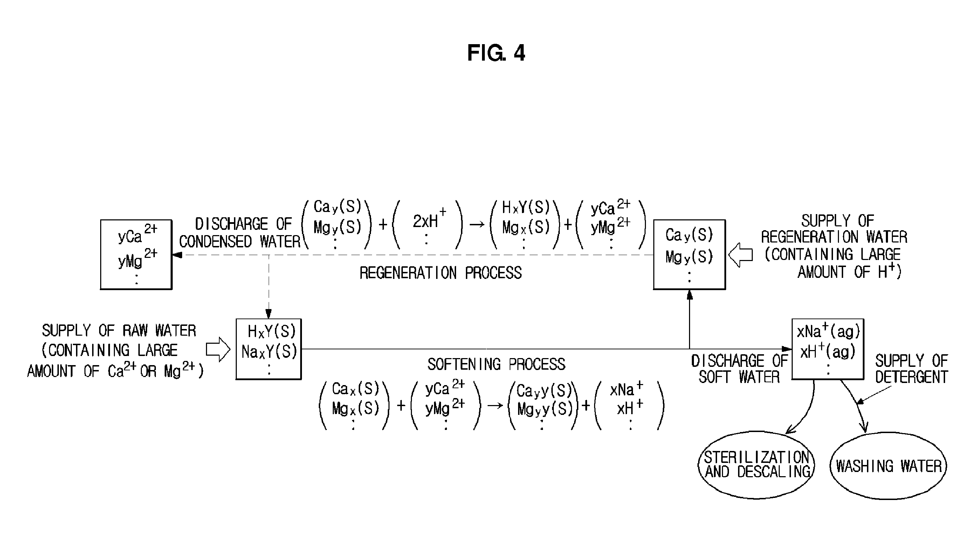

FIG. 2 is a view showing a softening process of the softening apparatus 100 according to the embodiment, FIG. 3 is a view showing a regeneration process of the softening apparatus 100 according to the embodiment, and FIG. 4 is a view showing the softening and regeneration processes performed in FIGS. 2 and 3 as a chemical reaction formula.

Referring to FIG. 2, when raw water is introduced into the softening unit 120 through the inlet port 101, the raw water reaches the ion exchange body 121 filling the softening unit 120. When the raw water reaches the ion exchange body 121, a hardness component (calcium ions (Ca²⁺) or magnesium ions (Mg²⁺)) contained in the raw water is removed by the ion exchange body 121 and soft water is discharged through an outlet port 102a of the housing 110. That is, the raw water softening process is performed such that the hardness component of the raw water is adsorbed by the ion exchange body 121 and, at the same time, a positive ion component is separated from the ion exchange body 121.

The principle of ion exchange in the ion exchange body 121 is related to the structure of the ion exchange body 121. In one embodiment, the ion exchange body 121 includes a zeolite particle (Z) represented by structural formula 1.

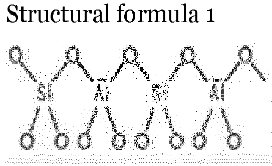

Referring to structural formula 1, the zeolite particle (Z) has silicon and aluminum as central atoms. The aluminum component of the zeolite particle (Z) partially has negative charges and, therefore, may adsorb positive ions having positive charges.

When raw water containing a hardness component (calcium ions (Ca²⁺) and magnesium ions (Mg²⁺)) is introduced to an initial zeolite particle (Z) coupled to hydrogen ions (H⁺) or sodium ions (Na⁺), therefore, ion exchange is performed between the hydrogen ions (H⁺) and the calcium ions (Ca²⁺) and the magnesium ions (Mg²⁺). In addition, ion exchange is performed between the sodium ions (Na⁺) and the calcium ions (Ca²⁺) and the magnesium ions (Mg²⁺).

Chemical reaction formulas 1 and 2 show a process in which the hardness component is adsorbed by the zeolite particle (Z).

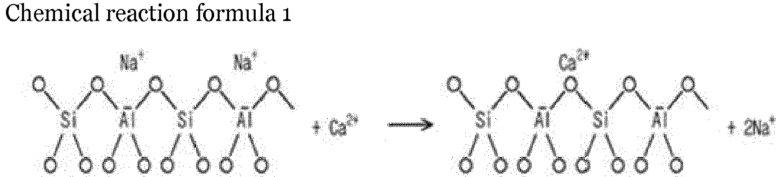

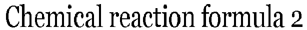

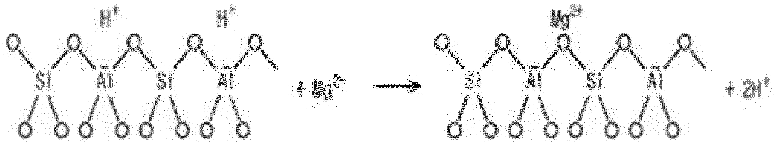

The initial zeolite particle (Z) may include sodium ions (Na⁺) or hydrogen ions (H⁺) based on kind thereof. However, the regeneration process is performed through ion exchange between high-concentration hydrogen ions (H⁺) generated during electrolysis of water and calcium ions (Ca²⁺) and magnesium ions (Mg²⁺). As the regeneration process and the softening process are repeatedly performed, ion exchange is repeatedly performed between the hydrogen ions (H⁺) and the calcium ions (Ca²⁺) and the magnesium ions (Mg²⁺). During ion exchange at the regeneration process and the softening process, the hydrogen ions (H⁺) are mainly intervened.

As concentration of hydrogen ions (H⁺) of water increases, pH of the water decreases and the water is acidified. Acid is corrosive. The ion exchange body 121 may be corroded due to such corrosiveness of acid. In the softening apparatus 100, zeolite is repeatedly regenerated and used for a long period of time. Consequently, zeolite stable against acid may be used as the ion exchange body 121.

When the softening process is performed for a predetermined amount of water, the regeneration process may be performed to remove impurities from the ion exchange body 121. That is, hard impurities may be removed from the ion exchange body 121 through the regeneration process such that the softening apparatus 100 is continuously usable.

Referring to FIG. 3, when raw water is introduced into the softening apparatus 100 through the inlet port 101 during the regeneration process, current is applied to the anode 131a and the cathode 131b of the regeneration unit 130. As a result, the raw water is electrolyzed to generate hydrogen positive ions.

When electric energy is applied to water such that the water is electrolyzed to perform an oxidation-reduction reaction, a reaction represented by chemical reaction formula 3 occurs at the anode 131a and a reaction represented by chemical reaction formula 4 occurs at the cathode 131b.

Chemical reaction formula 3 H₂O → ½O₂ + 2H⁺+ 2e⁻

Chemical reaction formula 4 2H₂O + 2e⁻ → H₂ + 2OH⁻

Referring to chemical reaction formulas 3 and 4, regeneration water having high concentration of hydrogen ions (H+) is generated from the anode 131a. When the regeneration water is supplied to the ion exchange body 121 provided in the vicinity of the anode 131a, calcium ions (Ca²⁺) and magnesium ions (Mg²⁺) adsorbed by the ion exchange body 121 are exchanged with the high-concentration hydrogen ions (H⁺) to regenerate the ion exchange body 121.

Meanwhile, a compound formed by coupling activated carbon (C) to zeolite particles (Z) may be used as the ion exchange body 121. Activated carbon (C) has a large specific surface area and high electric conductivity. When a compound formed by coupling activated carbon (C) to zeolite particles (Z) is used as the ion exchange body 121, therefore, the electrode 131 may have a large specific surface area.

That is, when activated carbon (C) is not coupled to zeolite particles (Z), hydrogen ions (H⁺) are mainly generated at the surface of the electrode. On the other hand, when activated carbon (C) is coupled to zeolite particles (Z), hydrogen ions (H⁺) may be generated in the vicinity of the activated carbon (C) in addition to at the surface of the electrode. As a result, regeneration water having high-concentration hydrogen ions (H⁺) may be obtained, thereby achieving more rapid regeneration of zeolite.

A softening and regeneration cycle as shown in FIG. 4 is derived from combination of the principles shown in FIGS. 2 and 3. In FIG. 4, a solid line indicates a softening process and a dotted line indicates a regeneration process.

Referring to FIG. 4, zeolite particles (Z) may have a form of HxY(s) or NaxY(s). When raw water containing a hardness component (Ca²⁺ or Mg²⁺) is supplied to zeolite particles (Z) of the softening unit 120, calcium ions (Ca²⁺) or magnesium ions (Mg²⁺) are adsorbed by the zeolite particles (Z) and, at the same time, a positive ion component, such as hydrogen ions (H⁺) or sodium ions (Na+), is separated from the ion exchange body 121. After completion of the softening process, therefore, soft water is discharged from the anode 131a side.

After completion of the softening process, a regeneration process may be periodically performed as needed. The regeneration process uses high-concentration hydrogen ions (H⁺) generated during electrolysis of water. That is, a large amount of hydrogen ions (H⁺) are generated from the anode 131a side during electrolysis of water. The hydrogen ions (H⁺) are exchanged with the calcium ions (Ca²⁺) or magnesium ions (Mg²⁺) adsorbed by the ion exchange body 121 to regenerate the zeolite particles (Z). After completion of the regeneration process, therefore, condensed water containing calcium ions (Ca²⁺) and magnesium ions (Mg²⁺) is discharged from the anode 131a side and alkali water containing a large amount of hydroxyl ions (OH⁻) is discharged from the cathode 131b side.

As a result, acid soft water containing hydrogen ions (H⁺) generated after completion of the softening process may be used to sterilize or descale another apparatus connected to the softening apparatus 100 or detergent may be supplied to the soft water through the detergent supply device 150 such that the soft water may be used as wash water. Meanwhile, the condensed water and the alkali water generated after completion of the regeneration process are discharged outside through a drain.

Next, construction and operation of a softening apparatus 100 including a heater 160 according to an embodiment will be described in detail. FIG. 5 is a view showing construction of a softening apparatus 100 including a heater 160 according to an embodiment, FIGS. 6A to 6C are views showing positions where the heater 160 may be installed in the softening apparatus, FIG. 7 is a graph showing the average adsorption amount of sodium ions (Na⁺) based on concentration of sodium chloride per temperature, and FIG. 8 is a graph showing a dissociation constant of water based on temperature.

Referring to FIG. 5, the softening apparatus 100 may further include a heater 160 in addition to the construction shown in FIG. 1 and a repeated description thereof corresponding to FIG. 1 will be omitted.

The heater 160 is an element to heat raw water supplied to the regeneration unit 130. During the regeneration process, the heater 160 may heat raw water supplied to the ion exchange body 121 such that the raw water is easily electrolyzed. When the temperature of the ion exchange body 121 is increased, calcium ions (Ca²⁺) or magnesium ions (Mg²⁺) may be easily separated from the ion exchange body 121. Consequently, a hardness component (Ca²+ or Mg²⁺) may be easily separated from the ion exchange body 121 using this principle.

More specifically, when temperature is changed from room temperature to high temperature, a dissociation constant of water is abruptly increased with the result that the water is easily electrolyzed. As the electrolysis result of the water, concentration of hydrogen ions (H⁺) is increased and, therefore, the hydrogen ions (H⁺) may be actively exchanged with the hardness component (Ca²⁺ or Mg²⁺) coupled to the ion exchange body 121.

Referring to FIGS. 6A to 6C, the heater 160 may be installed before the regeneration unit 130 and/or the softening unit 120 or in the vicinity of the regeneration unit 130 and/or the softening unit 120.

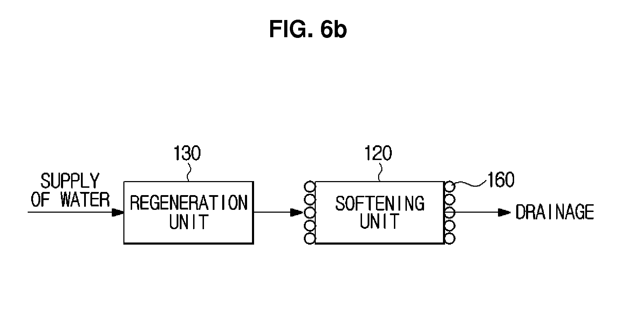

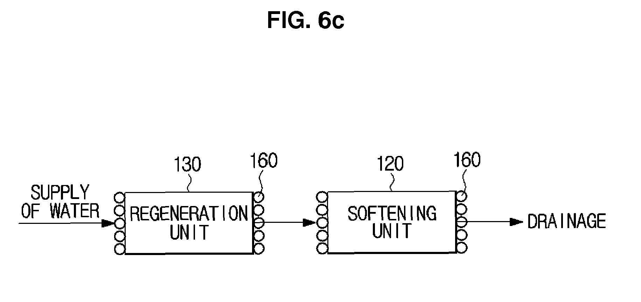

FIG. 6A shows that the regeneration unit 130 and the softening unit 120 are integrally formed and FIGS. 6B and 6C show that the regeneration unit 130 generates and supplies hydrogen ions (H⁺) to the softening unit 120 to perform regeneration. When the heater 160 is provided before the regeneration unit 130 and the softening unit 120 as shown in FIG. 6A, raw water heated by the heater 160 is supplied to the regeneration unit 130 such that the raw water is electrolyzed by the regeneration unit 130. Consequently, hydrogen ions (H⁺) may be more easily obtained on the anode 131a side and regeneration water having a large amount of hydrogen ions (H⁺) through electrolysis may be supplied to the ion exchange body 121 such that a hardness component (Ca²⁺ or Mg²⁺) is easily separated from the ion exchange body 121. In addition, even when the heater 160 is provided in the vicinity of the regeneration unit 130 and the softening unit 120 as shown in FIG. 6B, the above effects may be obtained.

As previously described, the heater 160 may be provided at the softening unit 120 or the regeneration unit 130. For example, as shown in FIG. 6C, the heater 160 may be installed in the vicinity of the softening unit 120. In this case, room-temperature raw water is supplied to the regeneration unit 130 such that the raw water is electrolyzed by the regeneration unit 130 and regeneration water obtained through electrolysis is supplied to the softening unit 120 such that the regeneration water is heated by the heater 160. The heated regeneration water may be supplied to the ion exchange body 121 such that a hardness component (Ca²⁺ or Mg²⁺) is easily separated from the ion exchange body 121.

Referring to FIG. 7, the adsorption amount of sodium is greater at high temperature than at low temperature. This is because motive power is thermodynamically increased to the heat at high temperature and, therefore, an ion separation property is increased.

The same principle may be applied to calcium ions (Ca²⁺) and magnesium ions (Mg²⁺). That is, when the ion exchange body 121 is regenerated using high-temperature regeneration water during the regeneration process, an ion separation property of calcium ions (Ca²⁺) and magnesium ions (Mg²⁺) is increased. Consequently, calcium ions (Ca²⁺) and magnesium ions (Mg²⁺) may be easily removed from the ion exchange body 121.

Referring to FIG. 8, a dissociation constant of water is abruptly increased when temperature is changed from room temperature to high temperature. Consequently, higher concentration of hydrogen ions (H⁺) may be obtained at high temperature and, therefore, regeneration may be easily achieved.

For example, a dissociation constant of water is 0.68 x 10⁻¹⁴ at 20°C. On the other hand, a dissociation constant of water is 33 x 10⁻¹⁴, which is about 48 times that at 20°C, at 85°C. When the heater 160 is installed such that high-temperature raw water is supplied to the ion exchange body 121 containing high concentration of hydrogen ions (H⁺) during the regeneration process, therefore, calcium ions (Ca²⁺) and magnesium ions (Mg²⁺) may be easily separated from the ion exchange body 121.

Next, a softening apparatus 100 including a storage tank 170 according to an embodiment will be described in detail. FIG. 9 is a view showing construction of a softening apparatus 100 including a storage tank 170 according to an embodiment.

Referring to FIG. 9, the softening apparatus 100 may further include a storage tank 170 in addition to the construction shown in FIG. 1 and a repeated description thereof corresponding to FIG. 1 will be omitted for the convenience of description.

The storage tank 170 stores soft water discharged from the softening unit 120 such that the soft water is supplied to the regeneration unit 130 during the regeneration process.

In the softening apparatus 100 shown in FIGS. 1 and 6, raw water containing a large amount of a hardness component (Ca²⁺ or Mg²⁺) is supplied to the regeneration unit 130. However, the regeneration process is performed such that high-concentration hydrogen ions (H⁺) generated during electrolysis of water are supplied to the ion exchange body 121. When the hardness component (Ca²⁺ or Mg²⁺) is supplied to the ion exchange body 121 during the regeneration process, therefore, ion exchange may be more effectively performed. In this embodiment, therefore, soft water is stored in the storage tank 170 during the softening process and then the soft water stored in the storage tank 170 is supplied to the regeneration unit 130 during the regeneration process. Consequently, the regeneration process may be more easily performed.

Next, a softening apparatus 100 including a softening unit 120 and a regeneration unit 130, which are separated from each other, according to an embodiment will be described in detail. FIG. 10 is a view showing a softening apparatus 100 including a softening unit 120 and a regeneration unit 130, which are separated from each other, according to an embodiment.

Referring to FIG. 10, the softening apparatus 100 includes the construction shown in FIG. 1. However, the regeneration unit 130 is installed before the softening unit 120. That is, the regeneration unit 130 and the softening unit 120 are separated from each other. Consequently, external appearances of the softening unit 120 and the regeneration unit 130 are defined by housings 110a and 110b.

A bead type zeolite compound is used as the ion exchange body 121. The ion exchange body 121 fills a gap between the inlet port 101 and the outlet unit 102 inside the housing 110a of the softening unit 120.

The electrode 131 includes a plate-shaped anode 131a and a plate-shaped cathode 131b provided in the housing 110b of the regeneration unit 130. The anode 131a and the cathode 131b are spaced apart from each other in a state in which the diaphragm 160 is disposed between the anode 131a and the cathode 131b.

Operation of the softening apparatus 100 is as follows. During the softening process of the softening apparatus 100, raw water having passed through the regeneration unit 130 installed before the softening unit 120 is introduced into the softening unit 120. At this time, electric power is not supplied to the electrode 131 of the regeneration unit 130. As a result, the raw water introduced into the regeneration unit 130 passes through the regeneration unit 130 and is introduced into the softening unit 120. The raw water introduced into the softening unit 120 is softened according to the same principle as shown in FIG. 2.

After the softening process is performed several times, it may be necessary to regenerate the ion exchange body 121 of the softening apparatus 100.

During the regeneration process of the ion exchange body 121, electric power is supplied to the electrode 131 of the regeneration unit 130 such that raw water introduced into the regeneration unit 130 is electrolyzed. When the raw water is electrolyzed, regeneration water having high concentration of hydrogen ions (H⁺) is obtained. The regeneration water is supplied to the ion exchange body 121 of the softening unit 120. The hydrogen ions (H⁺) of the regeneration water supplied to the ion exchange body 121 are exchanged with a hardness component (Ca²⁺ or Mg²⁺) adsorbed by the ion exchange body 121 to regenerate the ion exchange body 121.

Next, a softening apparatus 100 according to an embodiment will be described in detail. FIG. 11 is a view showing a cyclone type softening apparatus 100 according to an embodiment.

Referring to FIG. 11, the softening apparatus 100 is configured such that the softening unit 120 and the regeneration unit 130 are integrally formed, the housing 110 is designed to have a cyclone structure, and a power type zeolite compound fills the housing 110. In addition, the inlet port 101 is formed at one side of the housing 110 and the outlet port 102 is formed at the top of the housing 110.

Operation of the softening apparatus 100 with the above-stated construction is as follows. When raw water containing a hardness component (Ca²⁺ or Mg²⁺) is introduced into the housing 110 through the inlet port 101 during the softening process, cyclone is generated in the housing 110. As a result, zeolite particles (Z) sink and the water, which is lighter than the zeolite particles (Z), is softened and discharged through the outlet port 102 due to the difference in density between the zeolite particles (Z) and the water After the softening process is performed several times, it may be necessary to regenerate the ion exchange body 121 of the softening apparatus 100.

When raw water is introduced through the inlet port 101 of the housing 110 and electric power is supplied to the electrode 131 of the regeneration unit 130 to regenerate the ion exchange body 121, the raw water supplied to the regeneration unit 130 is electrolyzed and regeneration water having high concentration of hydrogen ions (H⁺) is obtained. The regeneration water obtained by the regeneration unit 130 is supplied to the ion exchange body 121 to regenerate the ion exchange body 121.

Next, a washing machine including the softening apparatus 100 shown in FIG. 1 will be described in detail. However, the softening apparatus 100 can be applied to any appliance, for example, a dishwasher or refrigerator, or a device that can benefit from softened water.

The washing machine may include a washing device, a softening apparatus 100, and a controller to control operation of the washing device and the softening apparatus 100. The softening apparatus 100 may include a regeneration unit 130 to generate regeneration water containing hydrogen ions (H⁺) and a softening unit 120, including an ion exchange body which is regenerated by the regeneration water, to convert raw water containing a hardness component into soft water containing hydrogen ions (H⁺). The washing machine may include all kinds of apparatuses, such as a washer and a dishwasher, using for washing. Hereinafter, a washer will be described in detail by way of example for the convenience of description.

FIG. 12 is a view showing a washing machine 200 including the softening apparatus 100 of FIG. 1 and FIG. 13 is a control block diagram of the washing machine 200 shown in FIG. 12. The washing machine 200 may include any one of the softening apparatuses 100 shown in FIGS. 1, 5, and 9 to 11. Hereinafter, the washing machine 200 including the softening apparatus 100 shown in FIG. 1 will be described in detail by way of example for the convenience of description.

Referring to FIGS. 12 and 13, the washing machine 200 includes a softening apparatus 100, channel units 141, 42, and 143 to guide soft water discharged from the softening apparatus 100, a plurality of valves 140 to allow or block flow of the soft water in the channel units 141, 42, and 143, an input unit 205 to allow input of a command to control the washing machine 200, a sensor unit 210 to determine regeneration time, a washing tub 290 to perform washing, a drive unit 220 to drive the washing tub 290 and the softening apparatus 100, and a controller 230 to control operation of the washing tub 290 and the softening apparatus 100. In addition, the washing machine 200 may further include a drain 190, which is a discharge passage of wash water discharged from the washing tub 290 and concentrated water and alkali water discharged from the softening apparatus 100 and a detergent supply device 150 to supply detergent to soft water generated by the softening apparatus 100.

The softening apparatus 100 includes a housing 110 having an inlet port 101 and an outlet port 102, a softening unit 120 having an ion exchange body 121 to convert raw water into soft water, and a regeneration unit 130 to electrolyze water to generate hydrogen ions (H⁺) and to supply the generated hydrogen ions (H⁺) to the ion exchange body 121 to regenerate the ion exchange body 121. Hereinafter, a repeated description of the softening apparatus 100 corresponding to FIG. 1 will be omitted for the convenience of description.

The input unit 205 is an element to allow input of a control command of the washing machine 200. The input unit 205 may be of a button type or a touch type. The washing machine 200 may be operated in a sterilization mode, a washing mode, and a regeneration mode. Correspondingly, the input unit 205 may include a sterilization mode input unit 205, a washing mode input unit 205, and a regeneration mode input unit 205.

The sensor unit 210 may be provided in the housing 110 of the softening apparatus 100 or around the outlet port 102a to determine regeneration time of the softening apparatus 100. For example, when the softening process is performed for a predetermined amount of water, the regeneration process may be performed to remove impurities from the ion exchange body 121. The sensor unit 210 senses a hardness component (Ca²⁺ or Mg²⁺) of soft water to determine regeneration time of the softening apparatus 100.

The sensor unit 210 may include at least one selected from among a hardness sensor, an electric conductivity sensor, a capacitive sensor, and a flow rate sensor. The hardness sensor senses a hardness component (Ca²⁺ or Mg²⁺) of soft water discharged from the softening unit 120. The electric conductivity sensor senses change in electric conductivity based on the hardness component (Ca²⁺ or Mg²⁺) of the soft water discharged from the softening unit 120. The flow rate sensor senses the amount of soft water treated by the softening unit 120 and outputs the sensing result to the controller 230.

The controller 230 controls the washing device to be operated in the sterilization mode, the washing mode, and the regeneration mode. After the softening and regeneration processes, the controller 230 controls flow of soft water and condensed water through the valves 140.

When a sterilization command is input through the input unit 205, the sterilization mode may be executed. When the washing machine 200 is operated in the sterilization mode, the softening unit 120 may generate soft water containing hydrogen ions (H⁺) such that the soft water is used to sterilize or descale the washing tub 290.

When a washing command is input through the input unit 205, the washing mode may be executed. When the washing machine 200 is operated in the washing mode, soft water discharged from the softening unit 120 may be mixed with detergent supplied from the detergent supply device 150 such that the mixture is supplied to the washing tub 290.

When a regeneration command is input through the input unit 205 or it is determined according to a predetermined criterion that the regeneration mode is to be executed, the regeneration mode may be executed. When the washing machine 200 is operated in the regeneration mode, the regeneration unit 130 may generate regeneration water containing hydrogen ions (H⁺) and supply the regeneration water to the softening unit 120 to regenerate the ion exchange body.

Hereinafter, a detailed description will be given of a regeneration time determination method of the washing machine 200 excluding a case in which the regeneration command is input through the input unit 205.

When the sensing result of the hardness sensor is output, the controller 230 may determine a hardness component (Ca²⁺ or Mg²⁺) of soft water according to the output signal of the hardness sensor. When the output of the hardness sensor reaches predetermined first reference hardness, the controller 230 may control the regeneration unit 130 to perform the regeneration process.

In addition, when the sensing result of the electric conductivity sensor is output, the controller 230 may determine a hardness component (Ca²⁺ or Mg²⁺) of soft water according to the output signal of the electric conductivity sensor. When the output of the electric conductivity sensor reaches predetermined second reference conductivity, the controller 230 may control the regeneration unit 130 to perform the regeneration process.

In addition, when the sensing result of the flow rate sensor is output, the controller 230 may check the amount of soft water treated by the softening unit 120 according to the output signal of the flow rate sensor. When the output of the flow rate sensor reaches predetermined third reference flow rate, the controller 230 may control the regeneration unit 130 to perform the regeneration process.

Next, a description will be given of a soft water and condensed water flow control process after the softening process and the regeneration process.

When the sterilization mode is input, soft water containing hydrogen ions (H⁺) discharge from the softening unit 120 is supplied to a position where sterilization or descaling is needed. As previously described, the soft water is acid water containing a large amount of hydrogen ions (H⁺). Consequently, the soft water may be introduced into the washing tub 290 through the second channel unit 142 to sterilize and descale the washing tub 290.

When the washing mode is input, soft water discharged from the softening unit 120 may be mixed with detergent supplied from the detergent supply device 150 such that the mixture is supplied to the washing tub 290. In this case, soft water containing a large amount of hydrogen ions (H⁺) may be used as wash water.

When the regeneration mode is input or it is determined that the regeneration mode is to be executed, electric power is applied to the electrode 131 to electrolyze water. Regeneration water containing high-concentration hydrogen ions (H⁺) may be obtained through electrolysis of water. Concentrated water discharged after completion of the regeneration process may be discharged outside through the drain.

Hereinafter, an operation method of the washing machine 200 will be described.

The operation method of the washing machine 200 includes an operation of supplying raw water containing a hardness component to the softening apparatus 100 to generate soft water containing hydrogen ions and an operation of providing the generated soft water to wash or sterilize the washing device. The operation of providing the generated soft water to wash the washing device may further include an operation of supplying detergent to the generated soft water and providing the soft water containing the detergent to the washing device to wash the washing device.

In addition, it may be necessary to periodically regenerate the ion exchange body of the softening apparatus 100 included in the washing machine 200 after the softening process is performed several times. Upon determining that the ion exchange body is to be regenerated, an operation of regenerating the softening apparatus 100 may be performed.

FIG. 14 is a flowchart showing an operation method of a washing machine 200 according to an embodiment. Hereinafter, the operation method of the washing machine 200 as a washer will be described in more detail by way of example.

Referring to FIG. 14, when raw water is supplied to the washing machine 200, the softening unit 120 softens the raw water into soft water. That is, a hardness component (Ca²⁺ or Mg²⁺) is removed from the raw water while the raw water passes through the softening unit 120 (310 and 320).

The hardness sensor senses hardness of the soft water discharged from the softening unit 120. At an early stage of the softening process, the hardness component (Ca²⁺ or Mg²⁺) is hardly sensed. As the softening process is performed several times, the hardness component (Ca²⁺ or Mg²⁺) accumulates in the ion exchange body 121. As a result, hardness having a predetermined value or more may be sensed. Consequently, the hardness sensor periodically senses the hardness of the soft water output from the softening unit 120 and outputs the sensing result to the controller 230 (330).

Upon receiving the output of the hardness sensor, the controller 230 determines an output value of the hardness sensor. Upon determining that the hardness component (Ca²⁺ or Mg²⁺) of the soft water discharged from the softening unit 120 has reached the predetermined first reference hardness, the controller 230 controls the regeneration unit to perform the regeneration process. On the other hand, upon determining that the hardness component (Ca²⁺ or Mg²⁺) of the soft water discharged from the softening unit 120 has not reached the predetermined first reference hardness, the controller 230 determines whether the sterilization mode has been input (340 and 350).

Upon determining that the hardness component (Ca²⁺ or Mg²⁺) of the soft water discharged from the softening unit 120 has reached the predetermined first reference hardness, it is determined that the regeneration process is to be performed. Consequently, electric power is supplied to the electrode 131 of the regeneration unit 130 such that raw water introduced into the regeneration unit 130 is electrolyzed. When the raw water is electrolyzed, hydrogen ions (H⁺) are generated and the hydrogen ions (H⁺) are exchanged with the hardness component (Ca²⁺ or Mg²⁺) coupled to the ion exchange body 121 to perform the regeneration process. When the regeneration process is completed, raw water is supplied to the softening unit 120 and the raw water is softened by the regenerated ion exchange body 121 (342, 344, 310, and 320). Concentrated water and alkali water generated during the regeneration process are discharged through the drain 190 via the third channel unit 143. In addition, prestored soft water may be supplied to perform the regeneration process as previously described with reference to FIG. 9.

Upon determining that the hardness component (Ca²⁺ or Mg²⁺) of the soft water discharged from the softening unit 120 has not reached the predetermined first reference hardness, the soft water discharged from the softening unit 120 is supplied to execute the sterilization mode or the washing mode (350).

Upon determining that the sterilization mode has been input through the input unit 205, the soft water discharged from the softening unit 120 is supplied to the washing tub 290 via the second channel unit 142 such that the soft water is used to sterilize and descale the washing tub 290 (350,352, and 354).

Upon determining that the sterilization mode has not been input through the input unit 205, it is determined that the washing mode has been input. Consequently, detergent is supplied to the soft water introduced into the first channel unit 141 through the detergent supply device 150. The soft water containing the detergent is supplied to the washing tub 290 such that the soft water is provided for washing (350, 360, 362, and 364).

The operation method of the washing machine 200 of embodiments of the invention is not limited to that shown in FIG. 14. The regeneration process may be performed after the washing process or the regeneration process may be directly performed through the input unit 205. That is, the above-described operation method of the washing machine 200 may include all processes within a scope easily changeable by those skilled in the art.

As is apparent from the above description, the softening apparatus and the washing machine according to the embodiments may have the following effects.

First, a zeolite compound that has been used to perform ion exchange may be regenerated using hydrogen ions (H⁺) generated using an electrochemical method such that the zeolite compound may be repeatedly used.

In addition, the zeolite compound may be continuously regenerated without supply of an additional regeneration agent, thereby improving economical efficiency.

Furthermore, hydrogen ions (H⁺) generated during a softening process may be used for sterilization and descaling, thereby executing a sterilization mode separately from a washing mode.

As discussed, embodiments of the invention can provide a softening apparatus comprising: a regeneration unit arranged to generate regeneration water containing hydrogen ions (H⁺), and a softening unit comprising an ion exchange body arranged to convert raw water containing a hardness component into soft water containing hydrogen ions (H⁺), wherein the ion exchange body is arranged to be regenerated by the regeneration water. The regeneration unit may be arranged to electrolyze water to generate the hydrogen ions (H⁺).

In some embodiments, the regeneration unit and the softening unit are integrally formed in one housing. In some such embodiments, the regeneration unit comprises a housing forming an external appearance and an electrode provided in the housing, and the ion exchange body is coupled to one side of the electrode via a binder such that the regeneration unit and the softening unit are integrally formed.

In some embodiments, the regeneration unit comprises a cyclone type housing, a cylindrical anode provided in the housing, and a cathode disposed on a central axis of the anode, and the ion exchange body is disposed between the anode and the cathode.

In some embodiments, the hardness component of the raw water is adsorbed by the ion exchange body of the softening unit and, at the same time, hydrogen ions (H⁺) are separated from the ion exchange body to soften the raw water. This can be referred to as a softening mode.

In some embodiments, the hydrogen ions (H⁺) contained in the regeneration water supplied from the regeneration unit are adsorbed by the ion exchange body of the softening unit and, at the same time, a hardness component is separated from the ion exchange body to regenerate the ion exchange body. This can be referred to as a regeneration mode.

Embodiments of the invention can also provide a washing machine comprising: a washing device; a softening apparatus according to any one of the above mentioned embodiments; and a controller to control operation of the washing device and the softening apparatus.

In some embodiments, when the washing device is operated in a sterilization mode, the controller is arranged to control the softening unit to generate soft water containing hydrogen ions (H⁺) such that the soft water is used to sterilize or descale the washing device.

In some embodiments, the washing machine further comprises a detergent supply device arranged to supply detergent to the soft water discharged from the softening unit, wherein, when the washing device is operated in a washing mode, the controller is arranged to control the soft water discharged from the softening unit to be mixed with the detergent supplied from the detergent supply device such that the mixture is provided to the washing device.

In some embodiments, when the washing device is operated in a regeneration mode, the controller is arranged to control the regeneration unit to generate regeneration water containing hydrogen ions (H⁺) and to supply the regeneration water to the softening unit such that the ion exchange body is regenerated.

Although a few embodiments have been shown and described, it would be appreciated by those skilled in the art that changes may be made in these embodiments without departing from the principles of the invention, the scope of which is defined in the claims and their equivalents.

## Claims

1. A softening apparatus comprising:
a regeneration unit arranged to generate regeneration water containing hydrogen ions (H⁺), and
a softening unit comprising an ion exchange body arranged to convert raw water containing a hardness component into soft water containing hydrogen ions (H⁺), wherein the ion exchange body is arranged to be regenerated by the regeneration water.

2. The softening apparatus according to claim 1, wherein the regeneration unit is arranged to electrolyze water to generate the hydrogen ions (H⁺).

3. The softening apparatus according to claim 1 or 2, wherein the regeneration unit and the softening unit are integrally formed in one housing;
optionally wherein the regeneration unit comprises a housing forming an external appearance and an electrode provided in the housing, and the ion exchange body is coupled to one side of the electrode via a binder such that the regeneration unit and the softening unit are integrally formed.

4. The softening apparatus according to any one of claims 1 to 3, wherein
the regeneration unit comprises a cyclone type housing, a cylindrical anode provided in the housing, and a cathode disposed on a central axis of the anode, and
the ion exchange body is disposed between the anode and the cathode.

5. The softening apparatus according to any one of claims 1 to 4, wherein the hardness component of the raw water is adsorbed by the ion exchange body of the softening unit and, at the same time, hydrogen ions (H⁺) are separated from the ion exchange body to soften the raw water.

6. The softening apparatus according to any one of claims 1 to 5, wherein the hydrogen ions (H⁺) contained in the regeneration water supplied from the regeneration unit are adsorbed by the ion exchange body of the softening unit and, at the same time, a hardness component is separated from the ion exchange body to regenerate the ion exchange body.

7. The softening apparatus according to any one of claims 1 to 6, wherein the ion exchange body comprises at least one selected from a group consisting of zeolite, activated carbon, platinum (Pt), titanium (Ti), titanium oxide (TiO₂), carbon black, and manganese (Mn).

8. The softening apparatus according to any one of claims 1 to 7, further comprising a heater arranged to heat water supplied to at least one selected from between the softening unit and the regeneration unit.

9. A washing machine comprising:
a washing device;
a softening apparatus according to any one of claims 1 to 8; and
a controller to control operation of the washing device and the softening apparatus.

10. The washing machine according to claim 9, wherein, when the washing device is operated in a sterilization mode, the controller is arranged to control the softening unit to generate soft water containing hydrogen ions (H⁺) such that the soft water is used to sterilize or descale the washing device.

11. The washing machine according to claim 9 or 10, further comprising:
a detergent supply device arranged to supply detergent to the soft water discharged from the softening unit,
wherein, when the washing device is operated in a washing mode, the controller is arranged to control the soft water discharged from the softening unit to be mixed with the detergent supplied from the detergent supply device such that the mixture is provided to the washing device.

12. The washing machine according to any one of claims 9 to 11, wherein, when the washing device is operated in a regeneration mode, the controller is arranged to control the regeneration unit to generate regeneration water containing hydrogen ions (H⁺) and to supply the regeneration water to the softening unit such that the ion exchange body is regenerated.

13. An operation method of a washing machine comprising a washing device and a softening apparatus comprising an ion exchange body having hydrogen ions (H⁺) adsorbed thereby, the operation method comprising:
supplying raw water containing a hardness component to the softening apparatus to generate soft water containing hydrogen ions; and
providing the generated soft water to the washing device to wash or sterilize the washing device.

14. The operation method according to claim 13, further comprising supplying detergent to the generated soft water and providing the soft water containing the detergent to the washing machine to wash the washing device.

15. The operation method according to claim 13 or 14, further comprising heating the raw water containing the hardness component using a heater.
